Europäisches Patentamt

(19) European Patent Office

Office européen des brevets

(11) EP 0 724 886 A1

## (12) EUROPEAN PATENT APPLICATION
published in accordance with Art. 158(3) EPC

(43) Date of publication:
07.08.1996 Bulletin 1996/32

(21) Application number: 94930334.1

(22) Date of filing: 19.10.1994

(51) Int. Cl.⁶: **A61K 47/36**

(86) International application number:
PCT/JP94/01755

(87) International publication number:
WO 95/11043 (27.04.1995 Gazette 1995/18)

(84) Designated Contracting States:
AT CH DE DK FR GB IT LI NL SE

(30) Priority: 22.10.1993 JP 264917/93

(71) Applicant: TSUMURA & CO.
Chuo-ku, Tokyo 103 (JP)

(72) Inventors:
• NAGASAWA, Michio
Chiyoda-ku Tokyo 102 (JP)

• KIMURA, Takayoshi
Inashiki-gun Ibaraki 300-11 (JP)
• HAMADA, Yuji
Inashiki-gun Ibaraki 300-11 (JP)
• MAEDA, Shuei
Inashiki-gun Ibaraki 300-11 (JP)

(74) Representative: Woods, Geoffrey Corlett et al
J.A. KEMP & CO.
14 South Square
Gray's Inn
London WC1R 5LX (GB)

(54) **BASE FOR SUSTAINED-RELEASE PREPARATION, SUSTAINED-RELEASE PREPARATION, AND PROCESS FOR PRODUCING THE PREPARATION**

(57) The present inventions relate to a base comprising amorphous agar for a sustained-release pharmaceutical preparation, a production method of the sustained-release pharmaceutical preparation which method using the amorphous agar as the base, and a sustained-release pharmaceutical preparation in which the amorphous agar is combined as the base.

According to the present inventions, the sustained-release pharmaceutical preparation is provided by using a conventional method.

## Description

TECHNICAL FIELD

The present inventions relate to pharmaceutical preparations which can sustainedly release a drug contained in the digestive tract compared with commonly used pharmaceutical preparations, bases for such pharmaceutical preparations, and production methods for such pharmaceutical preparations.

BACKGROUND ART

Sustained-release pharmaceutical preparations can sustained-release drugs contained in them, thereby give several advantages: maintaining their efficacy for a long time, decreasing frequency of administration per day for patients, enhancing the compliance of the patients, or controlling the serum level of the drug which causes toxicity or side effects in patients to keep it under a certain level. Commonly used pharmaceutical preparations do not have such advantages.

In general, the sustained-release pharmaceuticals are produced by using the following methods in which the drugs are coated by water insoluble polymer, or the drugs are incorporated in a matrix obtained by mixture of drugs, vehicles, and oil soluble substance, for example, waxes, after they are mixed and molten.

However, such operations are complicated and it takes long time to establish a certain condition to produce the pharmaceutical preparations having constant quality level.

On the other hand, hydrogels of several compounds such as hydroxypropyl cellulose, hydroxypropylmethyl cellulose, methyl cellulose, carboxymethyl cellulose, and polyvinylpyrrolidone are known as base compounds for the Sustained-release pharmaceutical Preparations(see Japanese Patent laid-open Publications Nos. HEI 4-74137 and HEI 4-82826 and others). However, there are not described that agar is used as powdered additives in the sustained-release Pharmaceutical preparations for oral drug dosage forms concretely.

Furthermore, agar shows variety of gel properties which depend on their production process condition including extraction conditions and so forth or alga sources originally used. Japanese Patent laid-open Publication No. 60-241871 disclosed that the powdered agar can be used as a disintegrator for the disintegrating tablets containing Aspartame, which can solve rapidly.

DISCLOSURE OF THE INVENTION

The present inventions have an object to provide the sustained-release pharmaceutical preparations by using conventional production methods. The present inventors studied the release properties of a variety kind of agar, and they found that the sustained-release pharmaceutical preparations can be produced conveniently when an amorphous agar is combined in the form of powder in the pharmaceutical preparations. Then the present inventors completed the present invention.

Briefly, the present inventions include the following inventions.

(1) A base for a sustained-release pharmaceutical preparation comprising an amorphous agar.
(2) The base for a sustained-release pharmaceutical preparation according to (1), wherein the amorphous agar is powder.
(3) The base for a sustained-release pharmaceutical preparation according to (1), wherein the amorphous agar is Quick soluble agar.
(4) The base for a sustained-release pharmaceutical preparation according to (3), wherein the Quick soluble agar has dissolution ratio of 50 % or more when the agar is held at a concentration of 1.5 % at 70°C for 10 minutes.
(5) The base for a sustained-release pharmaceutical preparation according to (3), wherein the Quick soluble agar has dissolution ratio substantially 100 % when the agar is held at a concentration of 1.5 % at 80°C for 10 minutes.
(6) The base for a sustained-release pharmaceutical preparation according to (3), wherein the Quick soluble agar has the higher dissolution limit higher than 3 % when it is boiled under normal pressure.
(7) The base for a sustained-release pharmaceutical preparation according to (1), wherein the amorphous agar is pulverized by using a fine pulverizer so as to become amorphous.
(8) A base composition for a sustained-release pharmaceutical preparation comprising an amorphous agar and hydroxypropyl cellulose.
(9) The base composition for a sustained-release pharmaceutical preparation according to (8), wherein the amorphous agar is powder.
(10) The base composition for a sustained-release pharmaceutical preparation according to (8), wherein the amorphous agar is Quick soluble agar.
(11) The base composition for a sustained-release pharmaceutical preparation according to (8), wherein the amorphous agar is pulverised by using a fine pulverizer so as to become amorphous.

(12) A producing method for a sustained-release pharmaceutical preparation by using an amorphous agar as a base for the pharmaceutical preparation.

(13) The method according to (12), the amorphous agar is powder.

(14) The method according to (12), the amorphous agar is Quick soluble agar.

(15) The method according to (14), Quick soluble agar has dissolution ratio of 50 % or more when the agar is held at a concentration of 1.5 % in water at 70°C for 10 minutes.

(16) The method according to (14), wherein the Quick soluble agar has dissolution ratio Substantially 100 % when the agar is held at a concentration of 1.5 % in water at 80°C for 10 minutes.

(17) The method according to (14), wherein the Quick soluble agar has the higher dissolution limit higher than 3 % when it is boiled under normal pressure.

(18) The method according to (12), wherein the amorphous agar is pulverized by using a fine pulverizer so as to become amorphous.

(19) A producing method for a sustained-release pharmaceutical preparation by using both an amorphous agar and hydroxypropyl cellulose as a base for the pharmaceutical preparation.

(20) The method according to (19), the amorphous agar is powder.

(21) The method according to (19), the amorphous agar is Quick soluble agar.

(22) The method according to (21), Quick soluble agar has dissolution ratio of 50 % or more when the agar is held at a concentration of 1.5 % in water at 70°C for 10 minutes.

(23) The method according to (21), wherein the Quick soluble agar has dissolution ratio substantially 100 % when the agar is held at a concentration of 1.5 % in water at 80°C for 10 minutes.

(24) The method according to (21), wherein the Quick soluble agar has the higher dissolution limit higher than 3 % when it is boiled under normal pressure.

(25) The method according to (19), wherein the amorphous agar is pulverized by using a fine pulverizer so as to become amorphous.

(26) A sustained-release pharmaceutical preparation wherein an amorphous agar is combined as a base.

(27) The sustained-release pharmaceutical preparation according to (26), wherein the amorphous agar is powder.

(28) The sustained-release pharmaceutical preparation according to (26), wherein the amorphous agar is Quick soluble agar.

(29) The sustained-release pharmaceutical preparation according to (26), wherein the amorphous agar is pulverized by using a fine pulverizer so as to become amorphous.

(30) A sustained-release pharmaceutical preparation wherein both an amorphous agar and hydroxypropyl cellulose are combined as bases.

(31) A sustained-release pharmaceutical preparation according to (30), wherein the amorphous agar is powder.

(32) A sustained-release pharmaceutical preparation according to (30), wherein the amorphous agar is Quick soluble agar.

(33) A sustained-release pharmaceutical preparation according to (30), wherein the amorphous agar is pulverized by using a fine pulverizer so as to become amorphous.

In the present inventions, an amorphous agar is defined as the agar that does not show any crystallinity. It shows complete diffuse scattering without any diffraction peak showing crystallinity when the agar is analyzed by using a device for the powder X-ray diffraction. As examples of such amorphous agar, Quick soluble agar and the agar pulverized by using a fine pulverizer so as to become amorphous are given.

In the present specification, dissolution ratio described for Quick soluble agar is defined as follows.

$$\text{Dissolution ratio} = (\text{the strength of the gel dissolved}$$
$$\text{at a temperature/the strength of the gel completely dissolved}) \times 100\ (\%)$$

The Quick soluble agar used in the present inventions has the improved dissolution ratio compared to the commonly used agar. The agar has usually dissolution ratio of 50 % or more when the agar is held at the concentration of 1.5 % in water at 70°C for 10 minutes, and preferably dissolution ratio substantially 100 % when the agar is held at the same concentration in water at 80°C for 10 minutes. The agar can be dissolved in water at higher concentration, and its dissolution limit is more than 3 % when the agar is boiled under normal pressure, although that of the commonly used agar is equal to or less than 3 %. The agar has usually the dissolution limit of about 10 %, and it shows the amorphous property compared to the commonly used agar.

Various grades of Quick soluble agar are commercialized, and they can be used. As examples of commercialized agar, Ina agars UP-16, UP-26 and UP-37 produced by INA FOOD INDUSTRY CO., LTD. and the like are given.

As the amorphous agar, Quick soluble agar described above or agar which is pulverized by using the fine pulverizer, for instance, a vibrating rod mil, so as to become amorphous can be used. Any type of the fine pulverizer may be employed if it makes the agar amorphous by pulverizing. The device for the powder X-ray diffraction can be employed to confirm whether the agar becomes amorphous or not.

As examples of agar, a variety of agar such as the powdered agar, flakes agar, solid agar, square rod agar, and strings agar are given. Preferable is powdered agar because it has excellent fluidity, packing property, compressing property, and homogeneity when it is mixed with other component for the pharmaceutical preparations.

The sustained-release pharmaceutical preparations of the present invention can be produced by combining drugs with the amorphous agar described above, preferably as powders, to form pharmaceutical preparations such as granules, tablets, capsules, and troches according to the known methods. The drugs as active components in the pharmaceutical preparation are not limited.

In case the amorphous agar is combined as powder according to the present invention, the pharmaceutical preparation can be produced as follow. For tablets, the powdered amorphous agar is combined with the powdered drug to obtain the combined powder, and the combined powder is formed by using direct compression. As one of the other method, the powdered amorphous agar is combined with the powdered drug to obtain the granules by using dry granulation method to form the tablets by using compressing. When the components except the powdered amorphous agar are mixed and form the granules by wet granulation method, the powdered amorphous agar is combined with the granules to form the tablets by compressing. For granules, the powdered amorphous agar is combined with the drug to obtain the granules by dry granulation method. As one of the other method, the components for the pharmaceutical preparations except the powdered amorphous agar is mixed to obtain the granules by using wet granulation method and the powdered amorphous agar is combined. Then the granules are formed by using the same method as the above dry granulation method. The conventional agar can not give the pharmaceutical preparation to the sustained-release ability even if it is used as the powder as described above.

In general, the sustained-release pharmaceutical preparations of the present invention are produced with the known methods by using vehicles such as starches, lactose, sucrose, mannitol, carboxymethyl cellulose and inorganic salts.

The sustained-release pharmaceutical preparations of the present invention may contain binders, disintegrators, surfactants, lubricants, enhancers for the fluidity, flavoring agents, colorants, and perfumes. Concrete examples of they are shown in below.

[Binders]

Microcrystalline cellulose, crystalline cellulose carmellose sodium, methylcellulose, hydroxypropylcellulose, low substituted hydroxypropylcellulose,
hydroxypropylmethylcellulose 2208,
hydroxypropylmethylcellulose 2906,
hydroxypropylmethylcellulose 2910,
hydroxypropylmethylcellulose phthalate 200731,
hydroxypropylmethylcellulose phthalate 220824,
hydroxypropylmethylcellulose acetate succinate, carmellose sodium, ethylcellulose, carboxymethylethylcellulose,
hydroxyethylcellulose; wheat starch, rice starch, corn starch, potato starch; dextrin, pregelatinized starch, partly pregelatinized starch, hydroxypropyl starch, pullulan, polyvinylpyrrolidone K25, polyvinylpyrrolidone K30, aminoalkyl methacrylate copolymer E, aminoalkyl methacrylate copolymer RS, methacrylate copolymer L, methacrylate copolymer S, methacrylate copolymer LD, polyvinylacetal diethylamino acetate; polyvinyl alcohol; acasia, powdered acasia, gelatin, white shellac, tragacanth, purified sucrose, Macrogol 200, Macrogol 300, Macrogol 6000.

[Disintegrators]

Microcrystalline cellulose, methyl cellulose, low substituted hydroxypropyl cellulose, carmellose, carmellose calcium, carmellose sodium, crosscarmellose sodium; wheat starch, rice starch, corn starch, potato starch; partially pregelatinized starch, hydroxypropyl starch, sodium carboxymethyl starch, tragacanth.

[Surfactants]

Soybean lecithin, sucrose esters of fatty acid, polyoxyl 40 stearate, polyoxyethylene hydrogenated caster oil 100, polyoxyethylene hydrogenated caster oil 40, polyoxyethylene hydrogenated caster oil 50, polyoxyethylene hydrogenated caster oil 60,
polyoxyethylene [42]polyoxypropylene[67]glycol,
polyoxyethylene[54]polyoxypropylene[39]glycol,
polyoxyethylene[105]polyoxypropylene[5]glycol,
polyoxyethylene[160]polyoxypropylene[80]glycol,

polyoxyethylene[196]polyoxypropylene[67]glycol, sorbitan sesquioleate, sorbitan trioleate, sorbitan monostearate, sorbitan monopalmitate, sorbitan monolaurate, polysorbate 40, polysorbate 60, polysorbate 65, polysorbate 80, glyceryl monostearate, sodium lauryl sulfate, lauromacrogol.

[Lubricants]

Wheat starch, rice starch, corn starch; stearic acid, calcium stearate, magnesium stearate, silicon dioxide hydrate, light anhydrous silicic acid, synthetic aluminum silicate, dried aluminum hydroxide gel, talc, magnesium aluminometasilicate, dibasic calciumphosphate, anhydrous dibasic calciumphosphate, sucrose esters of fatty acids, waxes, hydrogenated vegetable oil, poly(ethyleneglycol).

[Enhancers for the fluidity]

Hydrated silicon dioxide, light anhydrous silicic acid, dried aluminum hydroxide gel, synthetic aluminum silicate, magnesium silicate.

The base for the sustained-release pharmaceutical preparations of the present invention can control the release properties in combination with cellulose derivatives such as hydroxypropylcellulose, hydroxypropylmethylcellulose, methylcellulose and carboxymethyl cellulose, or synthetic polymers such as aminoalkyl methacrylate copolymer and methacrylic acid copolymer. Particularly, when hydroxypropylcellulose is combined with the base of the present invention, they give the pharmaceutical preparation showing the zero-order release properties, and this pharmaceutical preparation is administrated once per day.

When the base alone is added to the sustained-release pharmaceutical preparations, the amount of the base of the present invention is 10 to 90 weight % of the whole weight of the pharmaceutical preparation, preferably 20 to 90 weight % of that. When the base is added to the sustained-release pharmaceutical preparations with the cellulose derivatives described above, the amount of the base, which is Quick soluble agar, is preferably 20 to 80 weight % of the pharmaceutical preparations, and the amount of the cellulose derivatives are preferably 20 to 80 weight % of that.

The agar employed in the present invention is provided as foods from 350 years ago up to now. Furthermore, the agar is described in the Japanese Pharmacopoeia, National formulary, and the European Pharmacopoeia. That is, the agar is complied with the standard of official papers, and it means that the safety of the agar is authorized. In addition, such agar is described in GRAS(Generally Recognized as Safe) list of the United States, and it also means that the agar is safe. The agar belongs to $A_1$ group as ADI(Acceptable Daily Intake) classified by FAO/WHO professional committee of food additives in food standard, and the dose of the agar per day is "not limited". In ADI, foods are classified five classes, $A_1$, $A_2$, B, $C_1$, $C_2$, from the most safe to not safe in order. The group $A_1$ comprises the foods which have determined ADI after their evaluation, and those are recognized that they are perfectly safe when they are eaten freely.

BRIEF DESCRIPTION OF DRAWINGS

Fig. 1 shows a dissolution profile of the drug from the pharmaceutical preparations of EXAMPLE 1. In Fig. 1, the closed circle is the tablet with Quick soluble agar, and the open circle is the tablet without Quick soluble agar.

Fig. 2 shows the dissolution profile of the drug from the pharmaceutical preparation of EXAMPLE 2. In Fig. 2, the closed circle is the tablet with Quick soluble agar, and the open circle is the tablet without Quick soluble agar.

Fig. 3 shows the dissolution profile of the drug from the pharmaceutical preparations of EXAMPLE 3. In Fig. 3, the closed circle is the tablet with Quick soluble agar, and the open circle is the tablet without Quick soluble agar.

Fig. 4 shows the dissolution profile of the drug from the pharmaceutical preparations of EXAMPLE 4. In Fig. 4, the closed circle is the tablet with Quick soluble agar, and the open circle is the tablet without Quick soluble agar.

Fig. 5 shows the dissolution profile of the drug from the pharmaceutical preparations of COMPARATIVE EXAMPLE 1. In Fig. 5, the closed circle is the tablet with agar, and the open circle is the tablet without agar.

Fig. 6 shows the dissolution profile of the drug from the pharmaceutical preparations of EXAMPLES 6 and 7, and COMPARATIVE EXAMPLE 2. In Fig. 6, the closed square shows data from EXAMPLE 6, the closed circle shows those from EXAMPLE 7, and the closed triangle shows those from COMPARATIVE EXAMPLE 2.

Fig. 7 shows the influence of pH on dissolution ratio of the tablets of EXAMPLE 6. In Fig. 7, the dot in the open square shows data obtained when the pharmaceutical preparation is placed in the purified water. The closed diamond shows those when the pharmaceutical preparation is placed in the first solution(pH 1.2) and the open circle in the closed square shows those in the second solution(pH 6.8); both of the solutions are standardized in the Japanese Pharmacopoeia.

Fig. 8 shows a powder X-ray diffraction profile of the agar at each pulverized time by using measurements of the powder X-ray diffraction.

Fig. 9 shows the effect for the sustained-release which is given by the pulverized amorphous agar. In Fig. 9, the open circle shows the data from the tablet containing untreated agar and the closed triangle shows those form the tablet containing the agar pulverized for 20 minutes, and the closed circle shows those form the tablet containing the agar pulverized for 60 minutes.

BEST MODE FOR CARRYING OUT THE INVENTION

The present invention is explained herein below by using EXAMPLES. However, the present invention is not limited to them.

In the EXAMPLES described in below, Ina agar UP-37(INA FOOD INDUSTRY CO., LTD.) is used as Quick soluble agar, and it has the properties as follows.

Dissolution ratio held at the concentration of 1.5 % in water at 80°C for 10 minutes : 100 %

the dissolution limit by boiling: approximately 10 % under normal pressure

the jelly strength(1.5 % aqueous solution, NIKKANSUI method): $700 \pm 30$ g/cm$^2$

solidifying point(natural cooling method): $38.0 \pm 1.0$°C

melting point(adverse method): $88.0 \pm 2.0$°C

sol viscosity(1.5 % aqueous solution, Type B rotational viscometer, 85°C sol): $6.0 \pm 1.0$ cp

(EXAMPLE 1)

Both nifedipine and polyvinylpyrrolidone are weighed so as to nifedipine/polyvinylpyrrolidone = 1/2. The solvent containing ethanol/methylene chloride = 2/1 is prepared. The solvent is added to the weighed both nifedipine and polyvinylpyrrolidone to mix to dissolve. The solution is added into lactose for agitating and kneading, and the weight ratio of the lactose/nifedipine is 7. The kneaded mixture is dried, and sieved suitably to prepare granules for mix. Quick soluble agar is added to the granules for mixing. Then the mixture is compressed to formulate flat tablets with 200 mg of the weight and 10 mm of diameter by using the compression machine.

The formulation and content amount are as follows.

|  | combined ratio(weight %) |
| --- | --- |
| nifedipine | 5 |
| polyvinylpyrrolidone | 10 |
| lactose | 35 |
| Quick soluble agar | 50 |
| total | 100 |

Dissolution test is performed under the following conditions. The tablets formulated according to the formulation described above, and the tablets formulated by compression according to the formulation without Quick soluble agar are used for the dissolution test.

condition of the dissolution test

test method: Method 2, which is referred to as the puddle method, described in the Japanese Pharmacopoeia, 12th edition

test solution: 2.5 % sodium lauryl sulfate aqueous solution

temperature of the test solution: $37 \pm 0.5$°C

rotation number: 50 rpm

detection method: UV, the detection wavelength 235 nm

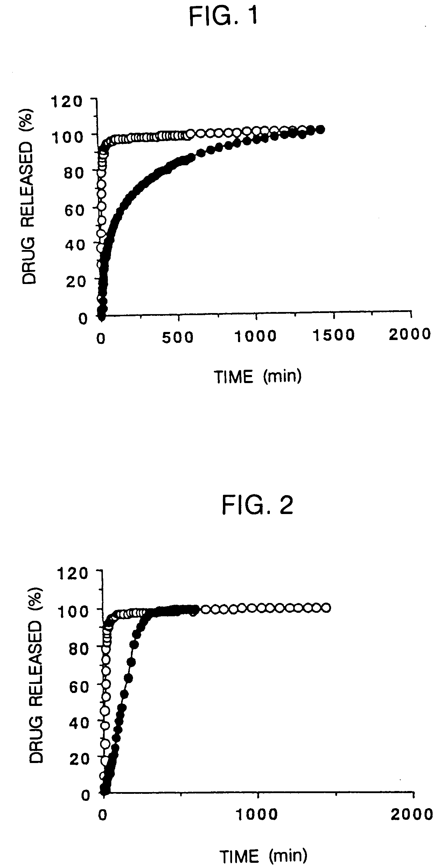

The result of the dissolution test is shown in Fig. 1. As shown in Fig. 1, the dissolution of the drug contained in the pharmaceutical preparation is suppressed when Quick soluble agar is added to the pharmaceutical preparation. It is demonstrated that the tablets which functions as the sustained-release pharmaceutical preparation is produced.

(EXAMPLE 2)

Both Quick soluble agar and hydroxypropylmethyl cellulose (HPMC), which is referred to as HPMC herein below, are added to the granules for mix obtained in EXAMPLE 1. The mixture is mixed and formulated by using the compressing machine to obtain flat tablets with 200 mg of weight and 10 mm diameter.

The formulation and content amount are as follows.

|  | combined ratio(weight %) |
| --- | --- |
| nifedipine | 5 |
| polyvinylpyrrolidone | 10 |
| lactose | 35 |
| Quick soluble agar | 25 |
| HPMC | 25 |
| total | 100 |

Dissolution test is performed under the same condition employed in EXAMPLE 1. The tablets formulated according to the formulation described above, and the tablets formulated by compression according to the formulation of EXAMPLE 1 without Quick soluble agar are used for the dissolution test.

The result of the dissolution test is shown in Fig. 2. As shown in Fig. 2, it is demonstrated that the tablets which have zero-order releasing property is produced when Quick soluble agar are combined with HPMC. In this case, the dissolution ratio of the tablets reach 100 % after approximately 10 hours.

(EXAMPLE 3)

Both Quick soluble agar and hydroxypropyl cellulose, which is referred to as HPC herein below, are added to the granules for mixing obtained in EXAMPLE 1. The mixture is mixed and formulated by using the compressing machine to obtain flat tablets with 200 mg of weight and 10 mm diameter.

The formulation and content amount are as follows.

|  | combined ratio(weight %) |
| --- | --- |
| nifedipine | 5 |
| polyvinylpyrrolidone | 10 |
| lactose | 35 |
| Quick soluble agar | 25 |
| HPC | 25 |
| total | 100 |

Dissolution test is performed under the same condition employed in EXAMPLE 1. The tablets formulated according to the formulation described above, and the tablets formulated by compression according to the formulation of EXAMPLE 1 without Quick soluble agar are used for the dissolution test.

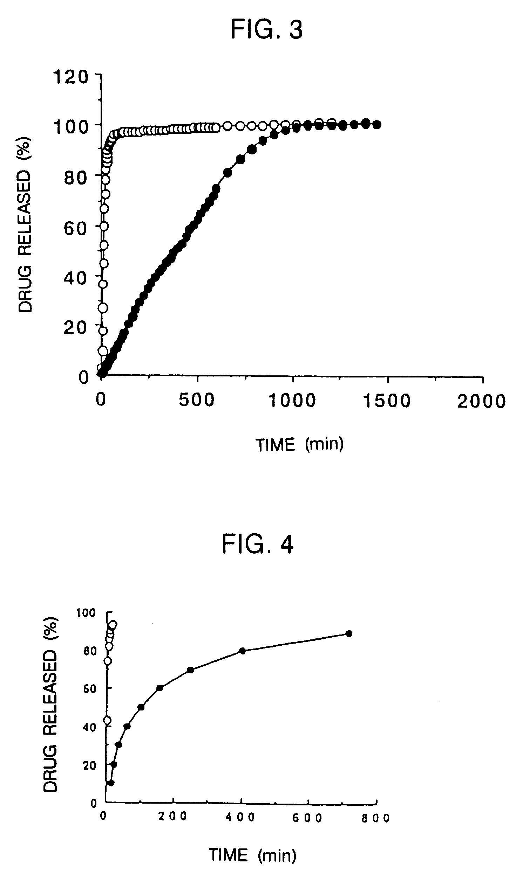

The result of the dissolution test is shown in Fig. 3. As shown in Fig. 3, it is demonstrated that the tablets which has zero-order releasing property is produced when Quick soluble agar is combined with HPC. In this case, the tablets for administrating to patients once per day are obtained.

(EXAMPLE 4)

Schizandrin as major component described in Japanese Patent Publication No. HEI 3-27530, microcrystalline cellulose(Avicel PH102, ASAHI CHEMICAL INDUSTRY), and crosscarmellose sodium(Ac-Di-Sol, ASAHI CHEMICAL INDUSTRY) are sieved by using suitable sieves. Quick soluble agar, light anhydrous silicic acid (Syloid 266, FUJI DEVISON CHEMICAL), and magnesium stearate(TAIHEI CHEMICAL INDUSTRIES) are weighed according to the following formulation. Then all compounds are mixed for 0.5 minutes, and compressed by using the compressing machine to obtain flat tablets with 140 mg of weight and 7 mm of diameter.

The formulation and content amount are as follows.

|  | combined ratio(weight %) |
|---|---|
| Schizandrin | 7 |
| Quick soluble agar | 66 |
| microcrystalline cellulose | 23 |
| crosscarmellose sodium | 3.5 |
| light anhydrous silicic acid | 0.2 |
| magnesium stearate | 0.3 |
| total | 100 |

Dissolution test is performed under the following conditions. Six tablets formulated according to the formulation described above, and six tablets formulated by Compression according to the formulation using lactose described in the Japanese Pharmacopoeia instead of Quick soluble agar are used for the dissolution test.

condition of the release test

test method: Method 2, which is referred to as the puddle method, described in the Japanese Pharmacopoeia, 12th edition
test solution: purified water
temperature of the test solution: $37 \pm 0.5°C$
rotation number: 100 rpm
test solution volume: 900 ml
detection method: UV, the detection wavelength $\lambda_1 = 252$ nm, $\lambda_2 = 310$ nm
$A_{max}$: 0.380

The result of the dissolution test is shown in Fig. 4. As shown in Fig. 4, the sustained-release pharmaceutical preparation of the present invention containing Quick soluble agar, which is the tablet, has improved sustained-release compared to the tablets containing lactose.

(COMPARATIVE EXAMPLE 1)

The tablets are formulated similar to the method of EXAMPLE 4 except that Ina agar S-6 (INA FOOD INDUSTRY CO., LTD.) which has the following properties is used instead of Quick soluble agar.

Properties of Ina agar S-6

Dissolution ratio held at the concentration of 1.5 % in water at 70°C for 10 minutes : 0 %
the jelly strength(1.5 % aqueous solution, NIKKANSUI method): $630 \pm 20$ g/cm$^2$
solidifying point(natural cooling method): $42.0 \pm 1.0°C$
melting point(adverse method): $87.0 \pm 1.0°C$
sol viscosity(1.5 % aqueous solution, Type B rotational viscometer, 85°C sol): $8.0 \pm 2.0$ cp

Dissolution test is performed by using the same condition as employed in EXAMPLE 4. The tablets formulated according to the formulation described above, and the tablets produced according to the process described above using the same amount of lactose described in the Japanese Pharmacopoeia instead of the agar described above are used for dissolution test.

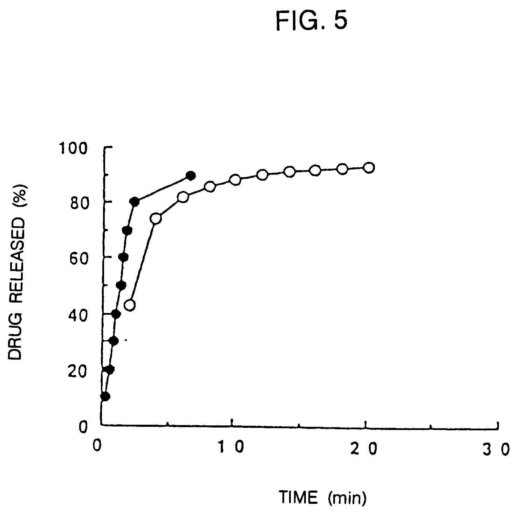

The result of the dissolution test is shown in Fig. 5. As shown in Fig. 5, the tablet containing the agar described above has rapidly releasing property compared to the tablet containing lactose.

(EXAMPLE 5)

Caffeine, which is easily soluble in water, is chosen as a model compound. The formulation of the sustained-release pharmaceutical preparation is studied.

The formulation and content amount are shown in below.

|  | combined ratio(weight %) |
| --- | --- |
| caffeine | 5 |
| Quick soluble agar | 47.5 |
| HPMC | 47.5 |
| total | 100 |

The powder mixture described above are formulated by using the compressing machine to obtain the flat tablets with 200 mg of weight and 10 mm diameter for the dissolution test. In the dissolution test performed here is the same as that in EXAMPLE 1 except purified water is employed as the test solution.

The result is shown in Table 1. In Table 1, the result obtained from the dissolution test in which the tablets containing 95 weight % of HPMC is shown too.

Table 1

| time(hr) | dissolution ratio(%) | |
| --- | --- | --- |
|  | agar/HPMC tablets | HPMC tablets |
| 1 | 40 | 44 |
| 2 | 61 | 71 |
| 3 | 81 | 88 |
| 4 | 88 | 99 |
| 5 | 95 | 99 |
| 7 | 100 | 100 |

As shown in Table 1, it is demonstrated that the tablets of the present invention, which contain both agar and HPMC, control the release of caffeine effectively compared to the tablets containing HPMC only. That is, the sustained-release pharmaceutical preparation of the present invention works effectively for the substances which are easily solved in water.

(EXAMPLE 6)

Both 30 g of nifedipine and 3 g of HPC are weighed and mixed. Ethanol is added to the mixture and stirred to solve them. The solution obtained is added to 120 g of microcrystalline cellulose to knead. Then the kneaded mixture is dried, and sieved by using the suitable sieve to obtain the granules for mix. After that, both 75 g of Quick soluble agar and 72 g of HPC are added to the granules for mixing to formulate the flat tablets with 300 mg of weight and 10 mm of diameter by using the compressing machine.

The formulation and content amount are shown in below.

|  | combined ratio(weight %) |
|---|---|
| nifedipine | 10 |
| HPC | 25 |
| microcrystalline cellulose | 40 |
| Quick soluble agar | 25 |
| total | 100 |

The tablets formulated as described above is employed in the dissolution test under the following conditions.

condition of the dissolution test

test method: Method 2, which is referred to as the puddle method, described in the Japanese Pharmacopoeia, 12th edition
test solution: 2 % sodium lauryl sulfate aqueous solution
temperature of the test solution: 37 ± 0.5°C
rotation number: 50 rpm
detection method: UV, the detection wavelength 254 nm

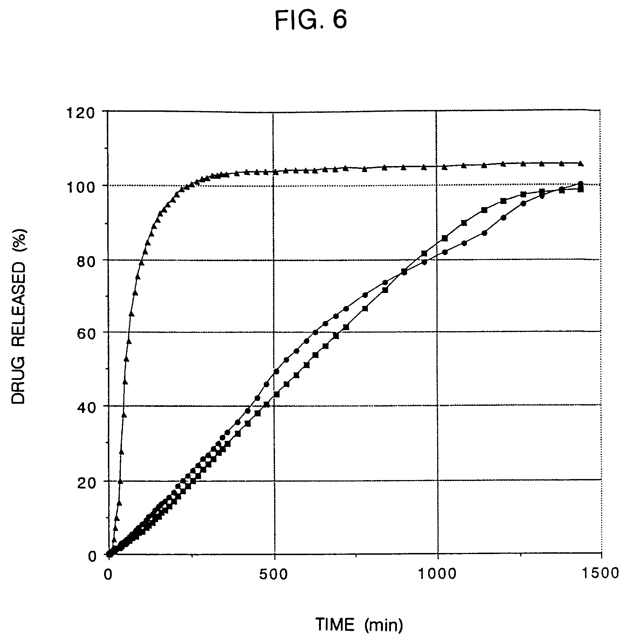

The result of the dissolution test is shown in Fig. 6. As shown in Fig. 6, it is demonstrated that the tablets which has zero-order releasing property is obtained when Quick soluble agar is combined with HPC. In this case, the tablets for administrating to patients once per day are obtained.

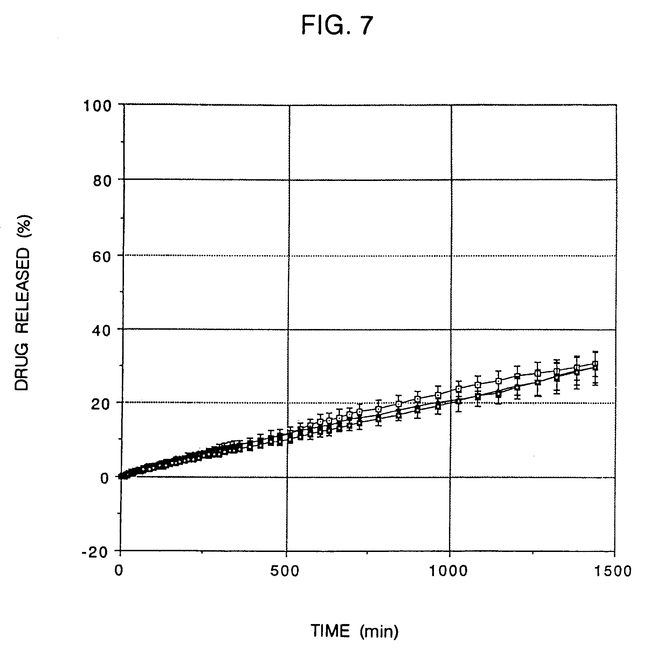

The tablets described above are subjected to other dissolution tests performed under the following conditions. In these tests, one of the solution comprising purified water, the first solution(pH 1.2) and the second solution(pH 6.8), both of which solutions are described in the Japanese Pharmacopoeia, is used as the test solution. The test is performed by using the puddle method, puddle rotation number 50 rpm, the temperature of the test solution is 37 ± 0.5°C. The results of the dissolution tests are shown in Fig. 7.

As shown in Fig. 7, the tablets of the present invention are not affected by pH of solution surrounding them.

(EXAMPLE 7)

Both 30 g of nifedipine and 3 g of HPC are weighed and mixed. Ethanol is added to the mixture and stirred to solve them. The solution obtained is added to 90 g of microcrystalline cellulose to knead. Then the kneaded mixture is dried, and sieved by using the suitable sieve to obtain the granules for mix. After that, both 40 g of Quick soluble agar and 37 g of HPC are added to the granules for mixing to formulate the flat tablets with 200 mg of weight and 10 mm of diameter by using the compressing machine.

The formulation and content amount are shown in below.

|  | combined ratio(weight %) |
|---|---|
| nifedipine | 15 |
| HPC | 20 |
| microcrystalline cellulose | 45 |
| Quick soluble agar | 20 |
| total | 100 |

The tablets formulated as described above is employed in the dissolution test under the same condition as that of EXAMPLE 6.

The result of the dissolution test is shown in Fig. 6.

As shown in Fig. 6, it is demonstrated that the tablets which has zero-order releasing property is obtained when Quick soluble agar is combined with HPC. In this case, the tablets for administrating to patients once per day are obtained.

(COMPARATIVE EXAMPLE 2)

Both 30 g of nifedipine and 3 g of HPC are weighed and mixed. Ethanol is added to the mixture and stirred to solve them. The solution obtained is added to 110 g of microcrystalline cellulose to knead. Then the kneaded mixture is dried, and sieved by using the suitable sieve to obtain the granules for mix. After that, both 30 g of Quick soluble agar and 27 g of HPC are added to the granules for mixing to formulate the flat tablets with 200 mg of weight and 10 mm of diameter by using the compressing machine.

The formulation and content amount are shown in below.

|  | combined ratio(weight %) |
|---|---|
| nifedipine | 15 |
| HPC | 15 |
| microcrystalline cellulose | 55 |
| Quick soluble agar | 15 |
| total | 100 |

The tablets formulated as described above is employed in the dissolution test under the same condition as that of EXAMPLE 6.

The result of the dissolution test is shown in Fig. 6.

As shown in Fig. 6, it is demonstrated that when the both Quick soluble agar and HPC are added in the tablets with their amount added of 15 weight % respectively, they do not give enough sustained-release.

(EXAMPLE 8)

(1) Preparation of amorphous agar by using fine pulverization

About 2 g of powdered Ina agar S-6(INA FOOD INDUSTRY CO., LTD.), which is used in COMPARATIVE ExAMPLE 1, is weighed. The agar is pulverised in a container for pulverization, Vibrating Sample Mill TI-200(CMT MFG. Co., Ltd.). Pulverizing times are 10, 20, 30, and 60 minutes, respectively. Their crystallinity is confirmed by using RINT 1100, which is powder X-ray diffraction measuring device(RIGAKU CORPORATION) for powder X-ray diffraction method.

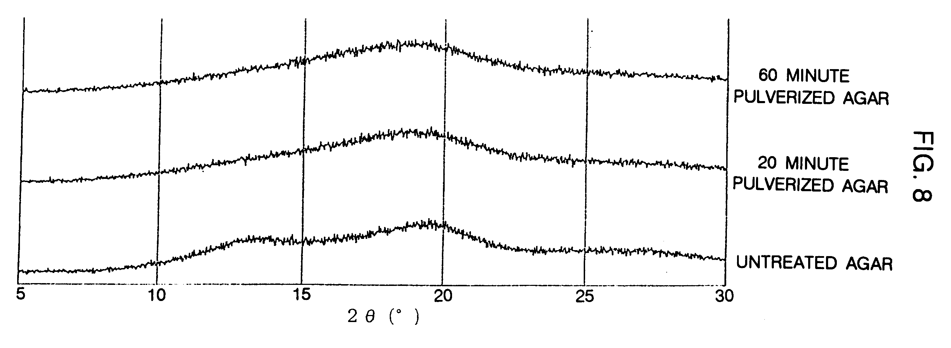

The results obtained in the X-ray diffraction profile are shown in Fig. 8.

In the untreated agar, diffraction peaks around $2\theta = 14°$ and $19°$ are observed and they shows crystallinity of the agar. However, after pulverizing, the peaks became broader. Accordingly, it is confirmed that Ina agar S-6 which is conventional agar becomes amorphous by fine pulverization. The diffraction profile of the agar made amorphous are the same as Quick soluble agar described above.

(2) Production of sustained-release tablets

Both 10 g of nifedipine and 3 g of HPC are weighed and mixed. Ethanol is added to the mixture and stirred to solve them. The solution obtained is added to 97 g of microcrystalline cellulose to knead. Then the kneaded mixture is dried, and sieved by using the suitable sieve to obtain the granules for mix. After that, 110 g of either amorphous agar obtained in (1) by fine pulverization or untreated agar(Ina agar S-6) is added to the granules for mixing to formulate the flat tablets with 220 mg of weight and 10 mm of diameter by using the compressing machine.

The formulation and content amount are shown in below.

|  | combined ratio(weight %) |
| --- | --- |
| nifedipine | 4.5 |
| HPC | 1.4 |
| microcrystalline cellulose | 44.1 |
| agar | 50.0 |
| total | 100 |

The tablets formulated as described above is employed in the dissolution test under the following condition.

condition of the dissolution test

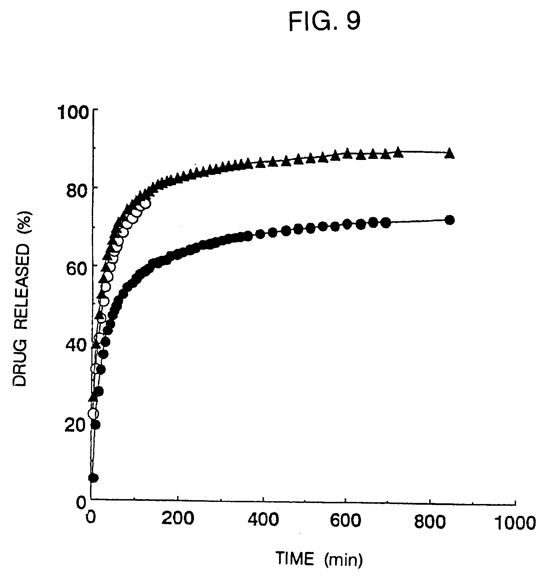

test method: Method 2, which is referred to as the puddle method, described in the Japanese Pharmacopoeia, 12th edition
test solution: purified water
temperature of the test solution: $37 \pm 0.5°C$
rotation number: 50 rpm
detection method: UV, the detection wavelength 254 nm
The result of the dissolution test for the tablets which contain either the pulverized agar for 20 or 60 minutes or untreated agar is shown in Fig. 9.
The tablets contain the pulverized agar for 20 minutes shows the almost same dissolution ratio as that of the tablets contain untreated agar. In contrast, the tablets contain the pulverized agar for 60 minutes shows clear sustained-release.
As a result, it is confirmed that the fine pulverization treatment gives the agar sustained-release ability.

INDUSTRIAL APPLICABILITY

The present invention gives the sustained-release pharmaceutical preparations by using convenient procedure.

**Claims**

1.  A base for a sustained-release pharmaceutical preparation comprising an amorphous agar.

2.  The base for a sustained-release pharmaceutical preparation according to claim 1, wherein said amorphous agar is powder.

3.  The base for a sustained-release pharmaceutical preparation according to claim 1, wherein said amorphous agar is Quick soluble agar.

4.  The base for a sustained-release pharmaceutical preparation according to claim 3, wherein said Quick soluble agar has dissolution ratio of 50 % or more when the agar is held at a concentration of 1.5 % at 70°C for 10 minutes.

5.  The base for a sustained-release pharmaceutical preparation according to claim 3, wherein said Quick soluble agar has dissolution ratio substantially 100 % when the agar is held at a concentration of 1.5 % at 80°C for 10 minutes.

6.  The base for a sustained-release pharmaceutical preparation according to claim 3, wherein said Quick soluble agar has the higher dissolution limit higher than 3 % when it is boiled under normal pressure.

7.  The base for a sustained-release pharmaceutical preparation according to claim 1, wherein said amorphous agar is pulverized by using a fine pulverizer so as to become amorphous.

8.  A base composition for a sustained-release pharmaceutical preparation comprising an amorphous agar and hydroxypropyl cellulose.

**9.** The base composition for a sustained-release pharmaceutical preparation according to claim 8, wherein said amorphous agar is powder.

**10.** The base composition for a sustained-release pharmaceutical preparation according to claim 8, wherein said amorphous agar is Quick soluble agar.

**11.** The base composition for a sustained-release pharmaceutical preparation according to claim 8, wherein said amorphous agar is fine pulverized by using a fine pulverizer so as to become amorphous.

**12.** A producing method for a sustained-release pharmaceutical preparation by using an amorphous agar as a base for said pharmaceutical preparation.

**13.** The method according to claim 12, said amorphous agar is powder.

**14.** The method according to claim 12, said amorphous agar is Quick soluble agar.

**15.** The method according to claim 14, Quick soluble agar has dissolution ratio of 50 % or more when the agar is held at a concentration of 1.5 % in water at 70°C for 10 minutes.

**16.** The method according to claim 14, wherein said Quick soluble agar has dissolution ratio substantially 100 % when the agar is held at a concentration of 1.5 % in water at 80°C for 10 minutes.

**17.** The method according to claim 14, wherein said Quick soluble agar has the higher dissolution limit higher than 3 % when it is boiled under normal pressure.

**18.** The method according to claim 12, wherein said amorphous agar is pulverized by using a fine pulverizer so as to become amorphous.

**19.** A producing method for a sustained-release pharmaceutical preparation by using both an amorphous agar and hydroxypropyl cellulose as a base for said pharmaceutical preparation.

**20.** The method according to claim 19, said amorphous agar is powder.

**21.** The method according to claim 19, said amorphous agar is Quick soluble agar.

**22.** The method according to claim 21, Quick soluble agar has dissolution ratio of 50 % or more when the agar is held at a concentration of 1.5 % in water at 70°C for 10 minutes.

**23.** The method according to claim 21, wherein said Quick soluble agar has dissolution ratio substantially 100 % when the agar is held at a concentration of 1.5 % in water at 80°C for 10 minutes.

**24.** The method according to claim 21, wherein said Quick soluble agar has the higher dissolution limit higher than 3 % when it is boiled under normal pressure.

**25.** The method according to claim 19, wherein said amorphous agar is pulverised by using a fine pulverizer so as to become amorphous.

**26.** A sustained-release pharmaceutical preparation wherein an amorphous agar is combined as a base.

**27.** The sustained-release pharmaceutical preparation according to claim 26, wherein said amorphous agar is powder.

**28.** The sustained-release pharmaceutical preparation according to claim 26, wherein said amorphous agar is Quick soluble agar.

**29.** The sustained-release pharmaceutical preparation according to claim 26, wherein said amorphous agar is pulverized by using a fine pulverizer so as to become amorphous.

**30.** A sustained-release pharmaceutical preparation wherein both an amorphous agar and hydroxypropyl cellulose are combined as bases.

**31.** A sustained-release pharmaceutical preparation according to claim 30, wherein said amorphous agar is powder.

**32.** A sustained-release pharmaceutical preparation according to claim 30, wherein said amorphous agar is Quick soluble agar.

**33.** A sustained-release pharmaceutical preparation according to claim 30, wherein said amorphous agar is pulverized by using a fine pulverizer so as to become amorphous.

## FIG. 1

## FIG. 2

## FIG. 3

## FIG. 4

## FIG. 5

FIG. 6

FIG. 7

# FIG. 8

FIG. 9

## INTERNATIONAL SEARCH REPORT

| International application No. |
| --- |
| PCT/JP94/01755 |

**A. CLASSIFICATION OF SUBJECT MATTER**

Int. Cl$^6$  A61K47/36

According to International Patent Classification (IPC) or to both national classification and IPC

**B. FIELDS SEARCHED**

Minimum documentation searched (classification system followed by classification symbols)

Int. Cl$^6$  A61K47/36

Documentation searched other than minimum documentation to the extent that such documents are included in the fields searched

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| A | JP, A, 4-74137 (Eisai Co., Ltd.), March 9, 1992 (09. 03. 92), Lines 5 to 8, lower left column, page 1, (Family: none) | 1-33 |
| A | JP, A, 4-82826 (Shin-Etsu Chemical Co., Ltd.), March 16, 1992 (16. 03. 92), Lines 5 to 9, lower left column, page 1, (Family: none) | 1-33 |

☐ Further documents are listed in the continuation of Box C.  ☐ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | |
| | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| December 5, 1994 (05. 12. 94) | December 27, 1994 (27. 12. 94) |

| Name and mailing address of the ISA/ | Authorized officer |
| --- | --- |
| Japanese Patent Office | |
| Facsimile No. | Telephone No. |

Form PCT/ISA/210 (second sheet) (July 1992)